# FASCICULE DE BREVET EUROPEEN

(11) **EP 0 637 264 B1**
(45) Date de publication et mention de la délivrance du brevet: **27.08.1997**
(21) Numéro de dépôt: 92906915.1
(22) Date de dépôt: 25.02.1992
(51) Int. Cl.: B01J 13/20, C12Q 1/02

(54) **GEL IONOTROPE DEFICIENT EN ENTITE IONIQUE DE GELIFICATION, PROCEDE DE PREPARATION D'UN TEL GEL ET UTILISATION DE CELUI-CI NOTAMMENT DANS UN PROCEDE D'ELABORATION DE VIN EFFERVESCENT**
Ionenarmes Gel als ionisierte Gelgesamtheit, Verfahren zu seiner Herstellung sowie seine Verwendung speziell in einem Verfahren zur Herstellung von Schaumwein
IONOTROPIC GEL LACKING A GELLING IONIC ENTITY, PROCESS FOR THE PREPARATION OF SUCH A GEL AND USE THEREOF ESPECIALLY IN A PROCESS FOR PRODUCING A SPARKLING WINE

(30) Priorité: 25.02.1991 FR 9102220
(43) Date de publication de la demande: 08.02.1995
(73) Titulaire: CHAMPAGNE MOET & CHANDON, 51205 Epernay (FR)
(72) Inventeur: RINN, Jean-Charles, F-16100 Cognac (FR); ROBILLARD, Bertrand, F-51200 Epernay (FR)
(74) Mandataire: Portal, Gérard
(86) Numéro de dépôt international: FR9200171
(87) Numéro de publication internationale: WO9214544

(56) Documents cités:
- FR-A- 2 633 937
- GB-A- 2 119 734
- GB-A- 2 153 780

## Description

La présente invention concerne essentiellement un gel ionotrope déficient en entité ionique de gélification, un procédé de préparation d'un tel gel et une utilisation de celui-ci notamment dans un procédé d'élaboration de vin effervescent tel que le champagne.

On sait qu'une catégorie de gels sont formés grâce à la fixation d'ions en certains sites précis de chaînes macromoléculaires, que l'on appelle sites de fixation ou sites de réticulation, formant ainsi des pontages entre ces chaînes. Ces ions, que l'on peut désigner sous l'expression générale "entité ionique de gélification" sont, par exemple, des cations polyvalents, généralement bi- ou trivalents, tels que l'ion calcium ou l'ion aluminium. Les gels ainsi formés sont parfois appelés "gels ionotropes". Parmi ces gels, on peut citer les alginates, les pectates, les carragénanes, la carboxyméthylcellulose et les chitosanes. Une description de ces gels a été faite en particulier par J. Klein et al. dans Angew. Makromol. Chem. (1979), vol. 76/77, n° 1141, p. 329-50 ; par K.D. Vorlop et al. dans Biotechnol. Lett. (1981), vol. 3, n° 1, p. 9-14 ; par H.J. Purz et al. dans Acta Polymerica (1985), vol. 36, n° 10, p. 569-574 et par R. Berger et al. dans Acta Biotechnol. (1988), vol. 8, n° 5, p. 401-405.

L'acide alginique et l'acide pectique, par exemple, sont constitués de chaînes polysaccharidiques et sont largement répandus dans le règne végétal.

Leur utilisation industrielle est bien connue, en particulier dans l'industrie alimentaire, en particulier pour réaliser des biotransformations.

Dans le cas de l'acide alginique, par exemple, les cations polyvalents, tels que l'ion calcium Ca²⁺, forment des pontages en certains sites précis des chaînes polysaccharidiques, correspondant à des séquences polyguluroniques, réalisant ainsi une structure en mailles. Ce type de structure réticulée est mis à profit, par exemple, dans l'immobilisation de micro-organismes, tels que bactéries ou levures, ou de macromolécules, telles que des enzymes.

Ainsi, pour certains procédés de fermentation utilisés dans les industries alimentaires par exemple, on a découvert l'intérêt d'utiliser des micro-organismes ou des enzymes, non plus à l'état libre, mais à l'état immobilisé dans des matériaux d'inclusion appropriés. Ces matériaux, grâce à leur structure en forme de réseau, retiennent les micro-organismes ou les enzymes, mais restent perméables aux substrats et aux produits de fermentation. Parmi les principaux avantages de cette technique, citons le fait qu'elle permet de travailler plus facilement en continu et qu'il est plus aisé de séparer les systèmes enzymatiques du milieu réactionnel (voir FR-A-2 320 349 INRA, le matériau d'inclusion étant une matrice de polyacrylamide ; FR-A-2 432 045 INRA, le matériau d'inclusion étant un polyacrylamide ou un alginate (revendication 4) ; l'Article dans la revue Pour la Science n° 146, décembre 1989, pages 20 à 21).

Selon l'une des techniques d'immobilisation de levures, celles-ci sont mises en suspension dans une solution aqueuse d'alginate de sodium. A partir de ladite suspension, on forme par exemple au moyen de buses de faible section des gouttelettes, que l'on fait ensuite tomber dans une solution de chlorure de calcium pour provoquer la formation d'un gel par réticulation ionique de l'alginate, sous forme de sphères gélifiées d'environ 2 à 3 mm de diamètre, communément appelées "billes". Ces billes sont ensuite rincées pour éliminer le chlorure de calcium en excès avant d'être utilisées telle quelles dans un procédé de fermentation, ou d'être conservées, soit dans un milieu aqueux approprié, soit, après séchage, pour une conservation de longue durée (FR 2 633 937).

De ce fait, les billes ainsi préparées, qui constituent des biocatalyseurs de fermentation, comportent une teneur importante en ions calcium, constituant principalement l'entité ionique de réticulation. D'une manière générale, on appelle "biocatalyseur" un système susceptible de réaliser une réaction biochimique, à partir d'un substrat, dans des conditions appropriées.

Par ailleurs, un problème très fréquent notamment dans les industries alimentaires, par exemple dans la préparation du boissons telles que les jus de fruit, le vin et le champagne, est celui de la précipitation sous forme de cristaux de certains composés tels que le bitartrate de potassium ou le tartrate de calcium. On a pu observer, par exemple dans le vin, que la solubilité de ces cristaux est largement dépendante du pH, du degré alcoolique, de la force ionique, de la température, mais aussi de la sursaturation du vin en ions calcium et surtout en ions potassium. Les risques de précipitation de tartrate de calcium apparaissent lorsque la concentration en ion calcium est supérieure à environ 80 mg/l. Or, dans certains vins, comme le champagne, cette concentration est comprise entre 60 et 110 mg/l environ. De plus, ce phénomène de cristallisation est particulièrement important dans certains processus oenologiques, tels que l'élaboration du champagne. En effet, lors de la seconde fermentation en bouteille, le degré alcoolique augmente, et des précipités apparaissent qui ne peuvent être éliminés par la suite, de façon relativement aléatoire, qu'à l'aide du remuage puis du dégorgement.

On comprend que la présence de dépôts cristallins est hautement préjudiciable à la qualité, notamment visuelle, des produits tels que le vin et surtout le champagne.

Dans le cas particulier du champagne, la présence de tels cristaux risque, en outre, de provoquer le phénomène dit de gerbage, c'est-à-dire l'expulsion brutale du liquide hors de la bouteille au moment de son ouverture.

Egalement, dans le cas de l'élaboration du champagne par la technique des levures immobilisées dans des billes de gel d'alginate de calcium, des cristaux de tartrate de calcium peuvent s'adsorber en surface des billes et les souder entre elles, formant ainsi une "plaque" de billes, ce qui pose un problème pour faire sortir ensuite ces plaques de la bouteille.

Enfin, la formation de ces cristaux est d'autant plus gênante et pernicieuse qu'elle se produit surtout dans le cas de la précipitation de tartrate de calcium, de façon lente et retardée, parfois même après que le produit ait été conditionné pour la vente.

Dans l'élaboration du champagne, ce phénomène de cristallisation a tendance à s'aggraver en raison de l'utilisation de billes de biocatalyseur contenant du calcium, comme c'est le cas des billes constituées d'alginate de calcium.

En effet, l'apport supplémentaire d'ions calcium dans le milieu à tendance à intensifier la précipitation de tartrate de calcium, car une partie non négligeable des ions calcium présents dans ces billes est libérée progressivement dans le vin sous l'action de phénomènes physico-chimiques.

Comme il a été dit plus haut, ce problème technique des dépôts cristallins n'est pas limité à la production de vin ou de champagne, mais il existe également de manière générale dans de nombreux procédés industriels faisant intervenir des liquides. On peut citer le cas de l'industrie des jus de fruits, notamment des jus de raisin, où l'on peut observer des dépôts de tartrates.

Une augmentation des précipitations tartriques est également observée à la suite du traitement de désacidification du vin, consistant à relever le pH par addition de carbonate de calcium.

On a tenté de remédier à ce phénomène de dépôts cristallins par exemple en induisant la précipitation de ces cristaux par divers moyens, tel qu'un passage au froid (S. FERENCZI et al., Bulletin, de l'O.I.V. 1982, N°613, p. 202), l'adjonction de germes ; ou bien en différant cette précipitation, par exemple par adjonction d'acide métatartrique (J. FARKAS et al., Kvasny Prum. 1982, vol. 28, N°8, p. 176-182 ; G. PARONETTO, Vignevini 1978, vol. 5, N°6-7, p 23-28).

Egalement, le document GB-A-2 153 780 DAMON décrit des lavages dans une solution saline, qui peut contenir du chlorure de calcium (exemples 1 et 2, page 4, lignes 22 à 32, où on n'élimine pas le calcium).

Cependant, après la réalisation d'une couche externe ou d'une peau externe (outer skin) en polylysine ou polyvinylamine, il est réalisé une dissolution du gel d'alginate en utilisant une solution de citrate de sodium 55 nM dans la même solution saline.

Ainsi, ce document démontre que pour éliminer le calcium contenu dans le réseau d'alginate, il est nécessaire d'utiliser une solution de citrate spécifique, qui redissout le gel, ce qui n'est évidemment pas réalisé par une simple solution saline dans les conditions de travail de DAMON.

Mais ces procédés ne sont pas réellement satisfaisants. En particulier, aucun ne règle le problème de la précipitation du tartrate de calcium. Par exemple, l'acide métatartrique est relativement instable. Il s'hydrolyse dans le temps et libère de l'acide tartrique. Son adjonction dans les vins aurait donc tendance dans le temps à aggraver le problème que l'on cherche à résoudre.

De plus, la concentration en cations calcium libres, dans certains milieux tels que le vin, augmente avec le temps. On sait en effet que certains ions, comme l'ion calcium, sont protégés par des substances polymériques (telles que des colloïdes) et ne seront libérés qu'à plus ou moins longue échéance.

On pourrait également envisager d'utiliser des résines synthétiques échangeuses de cations pour éliminer les cations en excès, mais cette méthode est prohibée en oenologie par la législation de nombreux pays, en particulier en France. De plus, en raison de la non-sélectivité de cette technique vis-à-vis de nombreux cations, une quantité importante de composants responsables de la qualité gustative risquerait d'être éliminée.

Ainsi, le problème technique de la précipitation de dépôts cristallins dans les boissons, notamment les boissons fermentées telles que le vin ou le champagne, n'a pu être résolu jusqu'à présent d'une manière satisfaisante.

La présente invention a donc pour but de résoudre le nouveau problème technique consistant en la fourniture d'une solution permettant d'éliminer, au moins en partie, les ions indésirables dans un milieu liquide donné.

Plus particulièrement, la présente invention a pour but de résoudre ledit problème technique en fournissant une solution permettant d'éliminer, au moins en partie, les ions responsables de la formation de dépôts cristallins dans les boissons, notamment les boissons fermentées, telles que la bière, le vin et le champagne.

La présente invention a encore pour but de résoudre le nouveau problème technique consistant en la fourniture d'une solution permettant de réduire l'apport d'ions responsables de précipitations de cristaux dans la mise en oeuvre de procédés ayant recours à l'emploi de biocatalyseurs constitués d'un matériau gélifié ionotrope, tel que l'alginate de calcium.

La présente invention a encore pour but de résoudre le nouveau problème technique consistant en la fourniture d'un matériau, formant notamment un biocatalyseur de fermentation, qui soit utilisable non seulement pour supprimer les risques de précipitations de dépôts cristallins, mais pouvant servir également dans les procédés enzymatiques ayant une activité enzymatique adaptée, notamment par la présence d'anions activateurs enzymatiques, ainsi que dans les procédés de reconnaissance ou de purification de matières organiques, comme par exemple dans le cas de la clarification de la bière pour éliminer les formations colloïdales.

La présente invention permet de résoudre pour la première fois les problèmes techniques énoncés précédemment, d'une manière satisfaisante, utilisable à l'échelle industrielle.

Car, il a été découvert, de façon tout à fait surprenante, que si on traitait les gels ionotropes, en particulier l'alginate de calcium, pour faire diminuer le taux de l'entité ionique de gélification, ces gels conservaient leur intégrité apparente, en particulier leur structure et leurs propriétés mécaniques, et pouvaient être utilisés dans diverses applications industrielles, notamment comme biocatalyseur de fermentation, sous forme de billes par exemple.

Ainsi, selon un premier aspect, la présente invention concerne un gel ionotrope solide, en particulier sous forme de billes, ou fixé sur un support approprié tel que grille ou filament, formé à partir d'un matériau gélifiable au moyen d'une entité ionique de gélification, caractérisé en ce que ledit gel est déficient en ladite entité ionique de gélification et présente des sites de fixation ionique résultant de l'absence de ladite entité ionique, lui conférant ainsi une affinité pour les ions capables de se fixer dans ledit gel sur lesdits sites de fixation inoccupés par l'entité ionique de gélification ; la teneur en entité ionique de gélification dans le gel étant inférieure à 0,75 fois la teneur maximale correspondant à la saturation dans ledit gel.

De préférence, la teneur en entité ionique de gélification dans le gel selon l'invention est inférieure à 0,5 fois, et de préférence encore comprise entre 0,005 fois et 0,05 fois la teneur maximale correspondant à la saturation dans ledit gel des sites de fixation de l'entité ionique de gélification.

Selon une variante de réalisation de l'invention, le matériau gélifiable précité est susceptible de s'écouler, et peut être avantageusement utilisé sous forme de gouttes que l'on gélifie en les mettant en contact avec une solution aqueuse contenant l'entité ionique de gélification précitée.

Selon une variante de réalisation particulière de l'invention, le matériau gélifiable précité est choisi parmi le groupe constitué par : les sels hydrosolubles de l'acide alginique et de l'acide pectique, notamment les sels de métal alcalin, tel que sodium ou potassium, ou le sel d'ammonium, un carragénane, notamment sous forme iota et kappa, le chitosane et la carboxyméthylcellulose.

Selon une variante de réalisation particulièrement avantageuse de l'invention, le matériau gélifiable précité est un matériau gélifiable par l'ion calcium. L'entité ionique de gélification étant ainsi constituée par l'ion calcium, ce qui permet d'obtenir, selon l'invention, un gel ionotrope appauvri en ions calcium, avide de cations.

Suivant une variante préférée, le gel selon l'invention est constitué par de l'alginate de calcium dont la teneur en ion calcium est inférieure à 1,5 mg/g de gel humide, de préférence inférieure à 1 mg/g, et de préférence encore elle est comprise entre 0,01 mg/g et 0,1 mg/g de gel humide.

Selon une autre variante de réalisation avantageuse de l'invention, le matériau gélifiable précité constitue un matériau d'inclusion de micro-organismes, notamment de micro-organismes de fermentation tels que les levures, ou de macromolécules telles que des enzymes, pour l'obtention d'un biocatalyseur gélifié déficient en entité ionique de réticulation, avide d'ions, en particulier d'ions calcium.

De préférence encore, ledit matériau d'inclusion ioniquement gélifiable est un matériau approprié compatible avec un milieu de fermentation, en particulier du domaine de l'oenologie, de préférence constitué par du vin, pour la production de vins effervescents, et notamment de champagne. Dans cette application au vin, en particulier au vin effervescent, notamment le champagne, il est préféré que la teneur en entité ionique de gélification soit inférieure ou égale à environ 0,30 fois la teneur maximale correspondant à la saturation.

Avantageusement, ledit matériau d'inclusion est choisi parmi le groupe constitué par les' sels alcalins ou d'ammonium de l'acide alginique ou pectique, de préférence l'alginate de sodium ou de potassium.

La présente invention, selon un deuxième aspect, concerne un procédé de préparation d'un gel solide ionotrope déficient en entité ionique de gélification, comprenant la gélification d'un matériau ioniquement gélifiable comportant des sites de fixation - ou sites de réticulation - sur lesquels se fixe ladite entité ionique de gélification, en produisant ainsi dans le gel ainsi formé la saturation des sites de fixation de ladite entité ionique, caractérisé en ce qu'après gélification par ladite entité ionique de gélification, on amène la teneur en entité ionique de gélification à un niveau inférieur à 0,75 fois celui de la teneur maximale correspondant à ladite saturation.

Avantageusement, selon une caractéristique préférée du mode de réalisation du procédé selon l'invention, on amène la teneur en entité ionique de gélification à un niveau inférieur à 0,5 fois celui de la teneur maximale correspondant à la saturation précitée, et de préférence encore il est compris entre 0,005 fois et 0,05 fois celui de ladite teneur maximale.

Selon un mode de réalisation particulièrement avantageux du procédé selon l'invention, on réalise la diminution de la teneur en entité ionique précitée du gel formé par gélification dudit matériau ioniquement gélifiable, par échange ionique, en particulier avec des protons, par exemple au moyen d'une solution aqueuse d'un acide que l'on met en contact avec le gel précité pour que se produise un échange ionique entre ladite entité ionique et le proton.

De préférence, le pH de ladite solution aqueuse acide est compris entre 1 et 3,5, et de préférence encore il est compris entre 2,5 et 3,2.

La nature de l'acide choisi n'est pas vraiment critique. En particulier, on pourra utiliser de l'acide chlorhydrique à une concentration correspondant à un pH convenable. Dans certains cas, en particulier lorsque le gel selon l'invention doit être utilisé dans un processus de fermentation par exemple en oenologie, on choisira de préférence un acide acceptable en alimentation tel que l'acide lactique. Toutefois, on choisira avantageusement un acide capable de former un complexe avec l'entité ionique de gélification, ce qui permet d'accélérer la réduction de la teneur en entité ionique de gélification du gel traité. Par exemple, en particulier lorsque l'entité ionique de gélification est l'ion calcium, on utilisera un diacide organique, dont les deux fonctions acides occupent de préférence les positions 1 et 4, tel que l'acide tartrique.

Les caractéristiques particulières décrites plus haut relativement au gel selon l'invention concernent également le présent procédé de préparation. En particulier, le matériau gélifiable est avantageusement susceptible de s'écouler en forme de gouttes que l'on transforme en billes gélifiées par action d'une solution aqueuse contenant l'entité ionique de gélification.

Selon une variante de réalisation particulière du procédé de préparation du gel selon l'invention, celui-ci est caractérisé en ce qu'on utilise comme matériau ioniquement gélifiable, un matériau compatible avec un milieu de fermentation, en particulier du domaine de l'oenologie, de préférence constitué par du vin, pour la production de vins effervescents, et notamment de champagne.

Un matériau ioniquement gélifiable préféré est constitué par un alginate alcalin, tel que l'alginate de sodium ou de potassium, ou l'alginate d'ammonium, l'entité ionique de réticulation étant constituée par l'ion calcium.

Suivant un mode de mise en oeuvre particulier du procédé de l'invention, le matériau ioniquement gélifiable constitue un matériau d'inclusion de micro-organismes, notamment de micro-organismes de fermentation tels que des levures, ou de macromolécules telles que des enzymes, pour l'obtention d'un biocatalyseur gélifié déficient en entité ionique de réticulation, avide d'ions, en particulier d'ions calcium.

La quantité de cellules de levure, tel que Saccharomyces cerevisiae, incluse dans le gel est du même ordre de grandeur que dans le cas d'immobilisation de cellules de levure dans des gels connus, par exemple cette quantité est comprise entre 100 millions et 600 millions de cellules de levure par millilitre de gel. Eventuellement, on peut aussi utiliser un gel à structure double couche comprenant un noyau dans lequel les cellules de micro-organismes sont incluses et une couche externe exempte desdites cellules.

Suivant une variante de réalisation pour laquelle la teneur en entité ionique de gélification doit être relativement faiblement abaissée, comme par exemple à moins d'environ 0,30 fois la teneur maximale à saturation, comme dans l'application en oenologie, l'abaissement peut être réalisé à une température voisine de la température ambiante, par exemple environ 20°C.

Suivant une variante du mode de réalisation précité particulièrement intéressante lorsque la teneur en entité ionique doit être très faible, l'abaissement de la teneur en entité ionique de gélification est effectué à une température comprise entre 4°C et 10°C, de préférence à environ 4°C.

Suivant une autre variante préférée du mode de réalisation précitée, lors de l'opération d'abaissement de la teneur en entité ionique de gélification, on réalise un apport en substrat nutritif dans le milieu contenant le gel ou biocatalyseur, en quantité juste nécessaire pour assurer la viabilité des micro-organismes, tels que les levures, inclus dans ledit gel ou biocatalyseur. Par exemple, dans le cas de Saccharomyces cerevisiae, la quantité de substrat apportée dans ce milieu sera d'environ 0,4 mg de saccharose par heure pour 300.10⁶ cellules.

Selon une caractéristique avantageuse du mode précité de mise en oeuvre du procédé selon l'invention, on maintient le pH de la solution acide précitée à une valeur d'au moins 2,7, lorsque le gel contient des cellules de micro-organismes, tels que des levures, afin de préserver l'activité desdites cellules.

Selon encore une autre variante de réalisation du procédé selon l'invention, après l'étape d'appauvrissement en entité ionique de gélification, on peut réaliser un nouvel échange ionique pour introduire un ion métallique en vue d'un emploi particulier, tel que l'activation enzymatique, ou la reconnaissance, la fixation ou purification d'un matériau organique comme des protéines ou des acides aminés. Ces ions métalliques sont de préférence choisis parmi le groupe consistant de magnésium, manganèse, zinc, potassium, fer, cuivre, calcium, cobalt, molybdène.

On observera que l'invention est particulièrement intéressante dans ces utilisations, car l'appauvrissement initial en entité ionique de gélification permet d'obtenir un milieu réactionnel propre et de régler, de manière très précise, la proportion d'addition de l'un ou l'autre des ions métalliques précédemment cités, d'une manière extrêmement reproductible et fiable. Il est ainsi possible de régler l'activité enzymatique car de nombreuses enzymes ont besoin de la présence d'un ion métallique pour leur activité et grâce à l'invention, cet ion métallique est présent en une quantité très précise, et stable grâce à l'inclusion dans le gel selon l'invention, puisque l'ion métallique participe à la structure chimique de ce gel.

La présence de cet ion métallique, en quantité très précise et fiable, permet également de fixer ou purifier des matériaux organiques, en particulier des protéines ou des acides aminés, car ces protéines ou ces acides aminés ont des sites ou des groupes se fixant préférentiellement sur des ions métalliques. On peut citer par exemple la reconnaissance d'histidine par l'ion cuivrique ou l'ion zinc.

Selon un troisième aspect, la présente invention concerne l'utilisation du gel selon l'invention, tel que précédemment défini, comme matériau destiné au piégeage d'ions, en particulier de cations.

Suivant un mode particulier de réalisation, on utilise le gel selon l'invention, en particulier sous forme de billes, dans l'industrie alimentaire, notamment dans l'industrie des jus de fruits et en oenologie, pour prévenir ou diminuer les risques de précipitation de cristaux, tels que le bitartrate de potassium et le tartrate de calcium.

Selon une variante particulière du mode de réalisation précité, on utilise un gel ionotrope, tel que l'alginate de calcium, déficient en entité ionique de gélification, tel que précédemment défini, de préférence sous forme de billes, dans un procédé de "prise de mousse", en particulier en bouteille selon la méthode dite "méthode champenoise", consistant en la deuxième fermentation d'un vin, tel qu'un vin de champagne, après adjonction de sucre pour l'obtention d'un vin effervescent. Avantageusement, le gel précité contient des levures, telles que Saccharomyces cerevisiae ou Saccharomyces bayanus. La concentration en levures est de préférence comprise entre 10⁸ et 6.10⁸ cellules de levure par millilitre de gel.

La quantité de gel selon l'invention contenant des levures, de préférence sous forme de billes, introduite par bouteille de 75 cl pour le procédé de "prise de mousse" est généralement d'environ 4 ml pour une concentration d'environ 3.10⁸ cellules de levure par millilitre de gel.

Est comprise également dans le champ de la présente invention l'utilisation dans le procédé de "prise de mousse" précité, de billes "classiques" de gel d'alginate de calcium, c'est-à-dire non déficientes en ions calcium, incluant des levures, auxquelles on ajoint des billes de gel selon l'invention n'incorporant pas de levure, ces dernières ayant pour seule fonction de piéger les cations indésirables, tels que les ions potassium et les ions calcium.

On remarquera toutefois que l'expérience a montré que l'utilisation dans le procédé de "prise de mousse" de billes de gel d'alginate de calcium déficient en calcium, selon l'invention, incluant des levures, présentait un avantage important inattendu sur le plan de la turbidité du vin effervescent obtenu. En effet, cette turbidité est bien plus faible dans le cas de l'utilisation des billes selon l'invention que dans celui des billes classiques non déficientes en ion calcium. Avec les billes selon l'invention, et en particulier celles ayant une teneur en calcium inférieure ou égale à environ 0,30 fois la teneur maximale à saturation, non seulement on évite les précipitations tartriques, mais également, l'évasion de levures hors des billes dans le vin est très fortement réduite voire inexistante. Cet avantage peut rendre inutile l'emploi, comme biocatalyseur, d'un gel à structure double couche tel que défini précédemment.

On observera, par ailleurs, que pour la mise en oeuvre "de la prise de mousse" du vin de champagne au moyen des billes selon l'invention, il suffit généralement que la teneur en calcium de ces billes soit abaissée à un taux d'environ 0,60 g/kg de billes humides, ce qui correspond à une teneur en calcium d'environ 0,30 fois la teneur maximale dans le gel, pour éviter tout risque de précipitation ultérieur de tartrate de calcium dans le vin.

Suivant un autre mode de réalisation de l'invention, on utilise le gel selon l'invention, en particulier sous forme de billes, ou fixé sur un support approprié, tel que grille ou filament, dans des procédés d'élimination de métaux lourds, tels que le plomb, le baryum, le cobalt, le fer, le manganèse et le cuivre. Avantageusement, le gel selon l'invention peut être utilisé, par exemple en remplissage de colonnes, pour le traitement en continu des eaux, notamment celui des effluents urbains ou industriels.

Selon un quatrième aspect, la présente invention concerne l'utilisation du gel selon l'invention, tel que précédemment défini dans un procédé enzymatique, en permettant ainsi de régler l'activité enzymatique, ledit gel comprenant alors une teneur déterminée en ion activateur enzymatique. Un tel ion activateur enzymatique est en particulier choisi parmi le groupe consistant de magnésium, de manganèse, de zinc, de potassium, de fer, de cuivre, de calcium, de cobalt, de molybdène.

Enfin, selon un cinquième aspect, la présente invention concerne encore l'utilisation du gel selon l'invention, tel que précédemment défini dans un procédé de fixation ou de purification de matériaux organiques, en particulier de protéines ou d'acides aminés, ou encore un procédé de reconnaissance de tels protéines ou acides aminés. Dans ce cadre, le gel de l'invention contient alors une quantité prédéterminée d'ions métalliques de fixation, choisis parmi le groupe précédemment énoncé, permettant la fixation du matériau organique, en particulier des protéines ou des acides aminés.

D'autres buts, caractéristiques et avantages de l'invention apparaîtront clairement à lumière de la description explicative qui va suivre faite en référence à plusieurs exemples de l'invention donnés simplement titre à d'illustration et qui ne sauraient donc en aucune façon limiter la portée de l'invention.

### Exemple 1

### Préparation du gel selon l'invention déficient en entité ionique de gélification

On prépare de manière connue un gel sous la forme de billes à partir d'une solution d'alginate de sodium à 1,2% en poids obtenue en mélangeant 120 g d'alginate de sodium dans 10 l d'eau distillée.

On produit des gouttelettes en faisant s'écouler la solution d'alginate goutte à goutte, à l'aide d'un appareil classique de formation de gouttes pouvant comporter un tube vertical de diamètre intérieur de 0,5 mm environ, dans un bain de gélification qui consiste en une solution aqueuse de chlorure de calcium à environ 16% en poids. Lorsqu'une goutte tombe dans le bain de réticulation, il se forme une bille de gel d'environ 3 mm, l'ion calcium constituant une entité ionique de gélification de l'alginate de sodium, ce dernier contituant un matériau ioniquement gélifiable par échange des ions Na⁺ par les ions Ca²⁺, cette technique étant bien connue à l'homme de l'art.

Ces billes ainsi formées, sont agitées doucement dans la solution de CaCl₂ pendant un temps approprié pour compléter la réticulation. Ensuite, les billes sont tamisées et lavées plusieurs fois avec de l'eau déminéralisée.

Après quatre rinçages, la concentration en calcium dans ces billes brutes de réticulation est de l'ordre de 2 g/kg de billes humides.

Selon l'invention, on appauvrit ces billes en entité ionique de gélification, ici en calcium de la manière suivante :

Dans une cuve de 50 l équipée d'une agitation mécanique, on introduit 10 l de billes de gel préparées précédemment, et on fait passer en continu une solution aqueuse d'acide tartrique à pH compris entre 2,5 et 3,2, de préférence à 2,7, soit une concentration pondérale d'environ 0,05 % en acide tartrique, par exemple au moyen d'une arrivée par la partie inférieure de la cuve et une sortie par trop-plein supérieur. On règle le volume de solution acide à environ deux fois celui des billes, soit ici à 20 l environ. Le débit de la solution acide dans la cuve est réglée à environ 100 l/h, sa température peut être la température ambiante, c'est-à-dire comprise entre 18°C et 25°C.

Pendant le passage de cette solution, on maintient une agitation douce de manière à ne pas endommager les billes de gel.

D'après l'analyse des prélèvements effectués, on observe que la teneur en calcium des billes, qui était d'environ 2 g/kg avant traitement, chute assez rapidement pour atteindre environ 1 g/kg au bout de 2 h 30 et environ 0,4 g/kg au bout de 5 h.

Si l'on poursuit plus longtemps ce traitement dans les mêmes conditions, la diminution de la concentration en calcium dans les billes est plus lente : 0,3 g/kg à la 10e h, 0,1 g/kg à la 18e h et 0,05 g/kg environ au bout de 24 h.

On observe en outre qu'au cours de l'échange d'ions précité, le diamètre des billes décroît sensiblement. Cette diminution est d'environ 25 % au bout de 24 h, pour des billes mesurant environ 3 mm avant traitement.

On remarque enfin, et cela de façon inattendue, que la structure du gel formant ces billes, en dehors de l'effet de contraction observé, ne semble pas avoir été modifié par le procédé décrit. En particulier, leurs propriétés mécaniques sont conservées, ce qui les rend aptes, en particulier à des utilisations industrielles, telles que celles précédemment décrites.

### Exemple 2

### Préparation d'un gel selon l'invention, constituant un biocatalyseur de fermentation

On prépare dans une première étape, des billes de gel d'alginate, comme à l'exemple 1, par gélification de gouttes de solution aqueuse d'alginate de sodium en présence d'une solution aqueuse de chlorure de calcium. Toutefois, dans le cas présent, les billes préparées ont une structure dite "double couche", c'est-à-dire formée d'un noyau constitué de gel d'alginate de calcium incluant des cellules de levure Saccharomyces cerevisiae, entourée d'une couche du même gel, mais sensiblement exempt de cellules de levure. Pour cela, on utilise un dispositif, tel que celui décrit dans le document DE-3 432 923 figure 5, constitué essentiellement de deux buses coaxiales verticales, disposées de façon que l'extrémité de la buse centrale soit légèrement plus basse que celle de la buse périphérique, et dont les dimensions sont telles qu'elles permettent l'écoulement goutte à goutte simultané de deux solutions aqueuses, l'une par la buse centrale, l'autre par la buse périphérique, la deuxième solution formant un film autour de la première. La solution arrivant par la buse centrale est une solution aqueuse d'alginate de sodium à 1,2 % en poids contenant 3.10¹¹ cellules par litre environ, celle arrivant par la buse périphérique est une solution aqueuse d'alginate de sodium de même concentration, mais ne contenant aucune cellule.

Comme à l'exemple 1, les gouttes tombent dans un bain de gélification consistant en une solution aqueuse de chlorure de calcium à environ 16 % en poids. En procédant ainsi, on immobilise les levures dans les billes de gel ainsi formées.

Dans l'étape suivante, consistant à faire diminuer le taux de calcium dans les billes, on opère également comme à l'exemple 1, si ce n'est que l'on effectue une alimentation supplémentaire d'une solution aqueuses de saccharose à 50 % à raison de 80 ml/h. De plus, les conditions opératoires sont en particulier les suivantes : le pH de la solution d'acide tartrique est compris entre 2,7 et 2,9, sa température est maintenue à 4°C.

Selon une première variante, on arrête le traitement par la solution acide dès que la teneur en calcium des billes atteint 0,1 g/kg environ, ce qui, dans les conditions opératoires précitées correspond à une durée de traitement d'environ 16 à 18 h.

Selon une deuxième variante permettant un traitement accéléré, lorsqu'il suffit, pour l'utilisation ultérieure des biocatalyseurs selon l'invention, par exemple dans le processus de prise de mousse, que la teneur en calcium dans les billes d'alginate soit d'environ 0,30 fois la teneur maximale à saturation, soit environ 0,65 g/kg de billes humides d'alginate, on traite avec une solution aqueuse d'acide tartrique à 1 % à une température voisine à la température ambiante, par exemple environ 20°C et le traitement ne dure alors qu'environ 1 h 30 min, ce qui est suffisant pour préserver les levures. De plus, dans ce cas, on observe que l'activité levurienne n'est pas lésée, même en l'absence d'alimentation supplémentaire en solution aqueuse de saccharose.

### Exemple 3

### Utilisation du biocatalyseur de l'exemple 2 pour réaliser la "prise de mousse" du champagne

On utilise des billes déficientes en calcium obtenues à l'exemple 2, contenant des cellules de Saccharomyces cerevisiae et titrant 0,1 g de calcium par kilogramme de billes humides. On introduit 4 ml de ces billes humides par bouteille de 750 ml contenant du vin sucré à raison de 24 g/l de saccharose. On ferme hermétiquement les bouteilles par une capsule, on les dispose en cave horizontalement de manière à laisser se produire la fermentation dite "prise de mousse".

Des prélèvements effectués après 13 jours de fermentation montrent qu'il se produit une diminution de la teneur en calcium dans le vin d'environ 10 mg/l, et une diminution du taux de potassium d'environ 30 mg/l. On n'observe, par ailleurs, aucune formation de cristaux dans les échantillons prélevés.

Après six semaines dans cette position, les bouteilles sont mises "sur pointe", c'est-à-dire le haut en bas pour permettre aux billes, de densité supérieure à celle du vin, de descendre vers le goulot, ce qui se produit généralement en quelques secondes. On opère ensuite selon la méthode champenoise classique, c'est-à-dire que l'on congèle au moyen d'une saumure le vin situé dans la partie inférieure du col de manière à emprisonner les billes dans un bouchon de glace ; celui-ci est ensuite éjecté après décapsulation de la bouteille.

Ainsi, grâce à l'emploi de ce biomatériau appauvri en calcium, il a été possible de stabiliser le vin vis-à-vis du calcium et/ou du potassium, tout en réalisant une fermentation alcoolique non perturbée. On observe, par ailleurs, de manière inattendue que le vin présente après la "prise de mousse" une moindre turbidité, tandis que dans le cas des procédés connus de "prise de mousse", avec des levures incluses ou non, subsiste toujours une très légère turbidité due en partie à la présence de colloïdes d'origine levurienne.

### Exemple 4

### Utilisation du biomatériau selon l'invention pour régler l'activité enzymatique ou pour reconnaître, fixer ou purifier des matériaux organiques tels que des protéines ou des acides aminés

On utilise les billes de gel préparées à l'exemple 1 comme matériau de départ pour la prépation d'un biomatériau contenant un i'on métallique en quantité réglée, de manière reproductible, par échange ionique classique. Cet ion métallique peut être choisi parmi le magnésium, le manganèse, le zinc, le potassium, le fer, le cuivre, le calcium, le cobalt, le molybdène, ou toute combinaison de ceux-ci.

Par exemple, on introduit une quantité réglée de cuivre en utilisant une solution aqueuse de sulfate de cuivre à 3% pour réaliser l'échange ionique entre les ions cuivriques et les protons du gel.

A cet effet, on introduit 100 ml de billes humides préparées à l'exemple 1 dans 200 ml de la solution de sulfate de cuivre. Le pH du milieu, qui était de 4,5 avant l'addition des billes, chute rapidement à 2,7, puis atteint 2,3 au bout de 2 h à température ambiante.

On obtient ainsi un biomatériau à activité enzymatique réglée qui peut être utilisé dans tout procédé enzymatique comme cela est bien apparent à l'homme de l'art.

On peut également reconnaître des protéines ou des acides aminés en utilisant également du zinc comme ion métallique. Ceci permet par exemple de reconnaître l'histidine, comme cela est également bien connu à l'homme de l'art.

Une autre application de l'invention dans le domaine de l'activation enzymatique consiste en une première étape à préparer des billes de gel ionotrope immobilisant une enzyme correspondant chacune à un cation activateur différent. Les procédés d'immobilisation d'enzymes sont bien connus de l'homme de l'art. On pourra se reporter notamment à l'ouvrage de M. MOO-YOUNG (ED.), "Bioreactor Immobilized enzymes and cells : fundamentals and applications" Elesevier Appl. Sci. Publish. (New York) 1988, notamment aux articles de A. Illanes et al., et de C. Dauner-Schütze et al.

Dans une seconde étape, l'entité ionique de réticulation est au moins en partie remplacée par des protons selon le procédé de la présente invention.

Au moment de l'utilisation de tels biomatériaux, il suffira de substituer les protons par le cation correspondant à l'enzyme que l'on souhaite activer.

### Exemple 5

### Essais comparatifs de traitement de l'eau et du vin pour l'élimination de métaux lourds

Dans le présent exemple, on place dans une première colonne (C₁) 4 ml de billes d'alginate déficientes en entité ionique de réticulation préparées à l'exemple 1, et dans une deuxième colonne (C₂) 4 ml de billes d'alginate de calcium non traitées. Une troisième colonne ne contient aucune bille, et sert de colonne témoin.

On fait passer 250 ml d'eau ou de vin au travers de chacune de ces trois colonnes, avec un débit de 85 ml/h pendant 4 h. On dose la concentration ionique avant et après traitement sur colonne. Les résultats figurent aux tableaux I et II ci-dessous :

**TABLEAU I**

| Concentration (ppm) | Eau non traitée | C₁ : billes "décalcifiées" | C₂ : billes "normales" |
|---|---|---|---|
| Mₙ | 260 | < 2 | 60 |
| Bₐ | 80 | < 1 | < 1 |
| Cu | 50 | < 5 | < 5 |
| Cₒ | 135 | < 10 | 30 |

**TABLEAU II**

| Concentration (ppm) | Vin non traité | C₁ : billes "décalcifiées" | C₂ : billes "normales" |
|---|---|---|---|
| Cₒ | 1,24 | 0,98 (-21 %) | 1,12 (-10 %) |
| Cu | 3,10 | 1,96 (-37 %) | 2,62 (-15,5 %) |
| Fₑ | 3,05 | 2,75 (-10 %) | 3,00 (-2 %) |

On constate que le traitement avec les billes décalcifiées selon l'invention (C₁) conduit à une diminution importante de la concentration en métaux lourds.

On observera également qu'avec l'emploi de billes dites normales, c'est-à-dire non décalcifiées, on observe une diminution de la concentration en métaux lourds, due probablement un effet d'absorption de ceux-ci à la surface des billes. Cependant, les billes décalcifiées selon l'invention procurent une amélioration inattendue de l'absorption des métaux lourds, qui est d'autant plus remarquable, qu'elle permet d'obtenir des concentrations extrêmement faibles en certains métaux lourds.

Il s'en suit que les gels selon l'invention peuvent être avantageusement utilisés comme moyen pour diminuer la teneur en métaux lourds de différents liquides. Notamment dans le domaine agro-alimentaire, il est particulièrement intéressant de pouvoir traiter des produits contaminés par des métaux lourds à la suite de traitements ou de la pollution de l'environnement.

## Revendications

1. Gel ionotrope solide, en particulier sous forme de billes ou fixé sur un support approprié tel que grille ou filament, formé à partir d'un matériau gélifiable au moyen d'une entité ionique de gélification, caractérisé en ce que ledit gel est déficient en ladite entité ionique de gélification et présente des sites de fixation ionique résultant de l'absence de ladite entité ionique, lui conférant ainsi une affinité pour les ions capables de se fixer dans ledit gel sur lesdits sites de fixation inoccupés par l'entité ionique de gélification ; la teneur en entité ionique de gélification étant inférieure à 0,75 fois la teneur maximale correspondant à la saturation dans ledit gel.

2. Gel ionotrope selon la revendication 1, caractérisé en ce que la teneur en entité ionique de gélification est inférieure à 0,5 fois, et de préférence encore comprise entre 0,005 fois et 0,05 fois la teneur maximale correspondant à la saturation dans ledit gel des sites de fixation de l'entité ionique de gélification.

3. Gel ionotrope selon la revendication 1 ou 2, caractérisé en ce que le matériau gélifiable précité est susceptible de s'écouler, et peut être avantageusement utilisé sous forme de gouttes que l'on gélifie en les mettant en contact avec une solution aqueuse contenant l'entité ionique de gélification précitée.

4. Gel ionotrope selon l'une des revendications 1 à 3, caractérisé en ce que le matériau gélifiable précité est choisi parmi le groupe constitué par les sels hydrosolubles de l'acide alginique et de l'acide pectique, notamment les sels de métal alcalin, tel que sodium ou potassium, ou le sel d'ammonium, un carragénane, notamment sous forme iota et kappa, le chitosane et la carboxyméthylcellulose.

5. Gel ionotrope selon l'une des revendications 1 à 4, caractérisé en ce que le matériau ionique gélifiable précité est un matériau gélifiable par l'ion calcium, l'entité ionique de gélification est ainsi constituée par l'ion calcium, en obtenant ainsi un gel ionotrope appauvri en ion calcium, avide de cations.

6. Gel ionotrope selon la revendication 5, caractérisé en ce qu'il est constitué par de l'alginate de calcium dont la teneur en ion calcium est inférieure à 1,5 mg/g de gel humide, de préférence inférieure à 1 mg/g, et de préférence encore comprise entre 0,01 mg/g et 0,1 mg/g de gel humide.

7. Gel ionotrope selon l'une des revendications 1 à 6, caractérisé en-ce que la teneur déficiente en entité ionique de gélification précitée est obtenue par échange ionique avec des protons, par exemple au moyen d'une solution aqueuse d'un acide, que l'on met en contact avec le gel précité pour qu'il se produise un échange ionique entre ladite entité ionique et le proton.

8. Gel ionotrope selon l'une des revendications 1 à 7, caractérisé en ce que le matériau ionique gélifiable précité constitue un matériau d'inclusion de micro-organismes, notamment de micro-organismes de fermentation tels que les levures, ou de macromolécules telles que des enzymes, pour l'obtention d'un biocatalyseur gélifié déficient en entité ionique de réticulation, avide d'ions, en particulier d'ions calcium.

9. Gel ionotrope selon la revendication 8, caractérisé en ce que le matériau d'inclusion ioniquement gélifiable est un matériau approprié compatible avec un milieu de fermentation, en particulier du domaine de l'oenologie, de préférence constitué par du vin, pour la production de vin effervescent, et notamment de champagne.

10. Gel ionotrope selon la revendication 9, caractérisé en ce que le matériau d'inclusion précité est choisi parmi le groupe constitué des sels alcalins ou d'ammonium de l'acide alginique ou pectique, de préférence l'alginate de sodium ou de potassium.

11. Gel ionotrope selon l'une des revendications 9 ou 10, caractérisé en ce que la teneur en entité ionique de gélification est inférieure ou égale à environ 0,30 fois la teneur maximale correspondant à la saturation.

12. Procédé de préparation d'un gel solide ionotrope déficient en entité ionique de gélification, comprenant la gélification d'un matériau ioniquement gélifiable comportant des sites de fixation, ou sites de réticulation, sur lesquels se fixe ladite entité ionique, en produisant ainsi dans le gel ainsi formé les saturation des sites de fixation de ladite entité ionique, caractérisé en ce qu'après gélification par ladite entité ionique de gélification, on amène la teneur en entité ionique de gélification à un niveau inférieur à 0,75 fois celui de la teneur maximale correspondant à ladite saturation.

13. Procédé selon la revendication 12, caractérisé en ce qu'on amène la teneur en entité ionique de gélification à un niveau inférieur à 0,5 fois celui de la teneur maximale correspondant à la saturation précitée, et de préférence encore à un niveau compris entre 0,005 fois et 0,05 fois celui de ladite teneur maximale.

14. Procédé selon la revendication 12 ou 13, caractérisé en ce qu'on réalise la diminution de la teneur en entité ionique précitée du gel formé par gélification du matériau ioniquement gélifiable, par échange ionique, en particulier avec des protons, par exemple au moyen d'une solution aqueuse d'un acide que l'on met en contact avec le gel précité pour que se produise un échange ionique entre ladite entité ionique et le proton.

15. Procédé selon la revendication 14, caractérisé en ce qu'on réalise la diminution de la teneur en entité ionique avec une solution aqueuse d'un acide dont le pH est compris entre 1 et 3,5, et de préférence compris entre 2,5 et 3,2.

16. Procédé selon la revendication 14 ou 15, caractérisé en ce qu'on utilise un acide acceptable en alimentation, tel que l'acide lactique.

17. Procédé selon la revendication 14, 15 ou 16, caractérisé en ce qu'on utilise un acide capable de former un complexe avec l'entité ionique de gélification, afin d'accélérer la réduction de la teneur en entité ionique de gélification, par exemple lorsque l'entité ionique de gélification est un ion calcium, on utilise un diacide organique dont ladite fonction acide occupe de préférence les positions 1 et 4, tel que l'acide tartrique.

18. Procédé selon l'une des revendications 12 à 17, caractérisé en ce que le matériau ioniquement gélifiable constitue un matériau compatible avec un milieu de fermentation, en particulier du domaine de l'oenologie, de préférence constitué par du vin, pour la production de vins effervescents, et notamment de champagne.

19. Procédé selon l'une des revendications 12 à 18, caractérisé en ce que le matériau ioniquement gélifiable constitue un matériau d'inclusion de micro-organismes, notamment de microorganismes de fermentation tels que les levures, ou de macromolécules telles que des enzymes, pour l'obtention d'un biocatalyseur gélifié déficient en entité ionique de réticulation, avide d'ions, en particulier d'ions calcium.

20. Procédé selon l'une des revendications 12 à 19, caractérisé en ce qu'on prépare un gel à structure double couche comprenant un noyau dans lequel des cellules de micro-organismes sont incluses et une couche externe exempte desdites cellules.

21. Procédé selon l'une-des revendications 19 et 20, caractérisé en ce que l'abaissement' de la teneur en entité ionique de gélification est effectué à une température comprise entre 4°C et 10°C, de préférence à environ 4°C.

22. Procédé selon l'une des revendications 19 à 21, caractérisé en ce que lors de l'opération d'abaissement de la teneur en entité ionique de gélification, on réalisé un apport en substrat nutritif dans le milieu contenant le gel ou le biocatalyseur, en quantité juste nécessaire pour assurer la viabilité des micro-organismes tels que les levures, inclus dans ledit gel ou biocatalyseur.

23. Procédé selon la revendication 22, caractérisé en ce que le micro-organisme précité est Saccharomyces cerevisiae, la quantité de substrat nutritif apportée dans ce milieu est d'environ 0,4 g de saccharose par heure pour 300.10⁶ cellules.

24. Procédé selon l'une des revendications 19 à 23, caractérisé en ce qu'on maintient le pH de la solution acide précitée à une valeur d'au moins 2,7, lorsque le gel contient des cellules de micro-organismes tels que des levures afin de préserver l'activité desdites cellules.

25. Procédé selon l'une des revendications 18 à 24, caractérisé en ce que la teneur en entité ionique de gélification est inférieure ou égale à environ 0,30 fois la teneur maximale correspondant à la saturation.

26. Procédé selon l'une des revendications 12 à 24, caractérisé en ce que, après l'étape d'appauvrissement en entité ionique de gélification, on peut réaliser un nouvel échange ionique pour introduire un ion métallique en vue d'un emploi particulier, tel que l'activation enzymatique, ou la reconnaissance, la fixation ou purification d'un matériau organique comme des protéines ou des acides aminés.

27. Procédé selon la revendication 26, caractérisé en ce que cet ion métallique est de préférence choisi parmi le groupe consistant de magnésium, manganèse, zinc, potassium, fer, cuivre, calcium, cobalt et molybdène.

28. Utilisation du gel tel que défini à l'une quelconque des revendications 1 à 11 ou tel qu'obtenu par le procédé selon l'une quelconque des revendications 12 à 27, comme matériau destiné au piégeage d'ions, en particulier de cations.

29. Utilisation du gel selon la revendication 28, en particulier sous forme de billes, dans l'industrie alimentaire, notamment dans l'industrie des jus de fruits, et oenologie, pour prévenir ou diminuer les risques de précipitation de cristaux, tels que le bitartrate de potassium et le tartrate de calcium.

30. Utilisation selon la revendication 28 ou 29, caractérisée en ce qu'on utilise un gel ionotrope, tel que l'alginate de calcium, déficient en entité ionique de gélification, de préférence sous forme de billes, dans un procédé de prise de mousse, en particulier en bouteille selon la méthode dite "méthode champenoise", consistant en la deuxième fermentation d'un vin, tel qu'un vin de champagne, après adjonction de sucre pour l'obtention d'un vin effervescent.

31. Utilisation selon la revendication 30, caractérisée en ce que le gel contient des levures telles que Saccharomyces cereviciae ou Saccharomyces bayanus.

32. Utilisation selon la revendication 31, caractérisée en ce que la quantité de gel d'alginate de calcium contenant des levures, de préférence sous forme de billes, introduite par bouteille de 75 cl pour le procédé de prise de mousse est généralement d'environ 4 ml pour une concentration d'environ 3.10⁸ cellules de levure par millilitre de gel.

33. Utilisation selon l'une des revendications 30 à 32, caractérisée en ce que du gel d'alginate de calcium, non déficient en ions calcium, incluant des levures, est utilisé en adjonction avec du gel n'incorporant pas de levure, déficient en ion calcium, ce dernier ayant pour seule fonction de piéger les cations indésirables tels que les ions de potassium et les ions calcium.

34. Utilisation selon l'une des revendications 29 à 33, caractérisée en ce que la teneur en entité ionique de gélification est inférieure ou égale à environ 0,30 fois la teneur maximale correspondant à la saturation.

35. Utilisation du gel ionotrope selon l'une des revendications 1 à 11, en particulier sous forme de billes ou fixé sur un support approprié, tel que grille ou filament, dans des procédés d'élimination de métaux lourds tels que le plomb, le baryum, le cobalt, le fer, le manganèse et le cuivre.

36. Utilisation selon la revendication 35, caractérisée en ce que le gel est utilisé, par exemple en remplissage de colonnes, pour le traitement continu des eaux, notamment celui des effluents urbains et industriels.

37. Utilisation du gel tel que défini à l'une des revendications 1 à 11 ou tel que préparé par le procédé selon l'une des revendications 12 à 27, dans un procédé enzymatique, en permettant ainsi de régler l'activité enzymatique, ledit gel comprenant alors une teneur déterminée en ions activateurs enzymatiques, en particulier choisi parmi le groupe consistant de magnésium, de manganèse, de zinc, de potassium, de fer, de cuivre, de calcium, de cobalt, de molybdène.

38. Utilisation du gel tel que défini à lune des revendications 1 à 11 ou tel que préparé par le procédé selon l'une des revendications 12 à 27, dans un procédé de fixation ou de purification d'un matériau organique, en particulier de protéines ou d'acides amines, ou dans un procédé de reconnaissance de tels protéines ou acides aminés.

39. Utilisation selon la revendication 38, caractérisée en ce que le gel ionotrope contient une quantité prédéterminée d'ions métalliques de fixation, choisis en particulier parmi le groupe consistant de magnésium, de manganèse, de zinc, de potassium, de fer, de cuivre, de calcium, de cobalt, de molybdène, permettant la fixation du matériau organique, en particulier des protéines ou des acides aminés.

## Claims

1. A solid ionotropic gel, in particular in the form of beads or bound to an appropriate support such as a grid or filament, formed from a material which can be gelled by means of an ionic gelling entity, characterized in that said gel is deficient in said ionic galling entity and has ionic binding sites resulting from the absence of said ionic entity, thereby giving it an affinity for ions which are capable of binding to said gel at said binding sites not occupied by the ionic galling entity; the proportion of ionic gelling entity being less than 0.75 times the maximum proportion corresponding to saturation in said gel.

2. The ionotropic gel according to claim 1, characterized in that the proportion of ionic gelling entity is less than 0.5 times and particularly preferably between 0.005 times and 0.05 times the maximum proportion corresponding to saturation, in said gel, of the binding sites for the ionic galling entity.

3. The ionotropic gel according to claim 1 or 2, characterized in that the above-mentioned gellable material is capable of flowing and can advantageously be used in the form of drops which are gelled by being brought into contact with an aqueous solution containing the above-mentioned ionic galling entity.

4. The ionotropic gel according to one of claims 1 to 3, characterized in that the above-mentioned gellable material is selected from the group consisting of the water-soluble salts of alginic acid and pectic acid, especially the alkali metal salts such as the sodium or potassium salts, or the ammonium salt, a carrageenan, especially in the iota and kappa form, chitosan and carboxymethyl cellulose.

5. The ionotropic gel according to one of claims 1 to 4, characterized in that the above-mentioned gellable ionic material is a material which can be gelled by the calcium ion, the ionic galling entity thus consisting of the calcium ion so as to obtain an ionotropic gel which is depleted in calcium ions and has an affinity for cations.

6. The ionotropic gel according to claim 5, characterized in that it consists of calcium alginate in which the proportion of calcium ions is less than 1.5 mg/g of moist gel, preferably less than 1 mg/g and particularly preferably between 0.01 mg/g and 0.1 mg/g of moist gel.

7. The ionotropic gel according to one of claims 1 to 6, characterized in that the above-mentioned deficient proportion of ionic gelling entity is obtained by ion exchange with protons, for example by means of an aqueous solution of an acid which is brought into contact with the above-mentioned gel so that ion exchange takes place between said ionic entity and the proton.

8. The ionotropic gel according to one of claims 1 to 7, characterized in that the above-mentioned gellable ionic material constitutes a material for the inclusion of micro-organisms, especially fermentation micro-organisms such as yeasts, or macromolecules such as enzymes, so as to obtain a gelled biocatalyst which is deficient in ionic crosslinking entity and has an affinity for ions, in particular calcium ions.

9. The ionotropic gel according to claim 8, characterized in that the ionically gellable inclusion material is an appropriate material compatible with a fermentation medium, in particular in the field of oenology and preferably consisting of wine, for the production of sparkling wine and especially champagne.

10. The ionotropic gel according to claim 9, characterized in that the above-mentioned inclusion material is selected from the group consisting of the alkali metal or ammonium salts of alginic or pectic acid, preferably sodium or potassium alginate.

11. The ionotropic gel according to one of claims 9 or 10, characterized in that the proportion of ionic gelling entity is less than or equal to about 0.30 times the maximum proportion corresponding to saturation.

12. A method of preparing a solid ionotropic gel deficient in ionic gelling entity, comprising the gelling of an ionically gellable material containing binding sites, or crosslinking sites, to which said ionic entity binds, thereby producing, in the gel thus formed, saturation of the binding sites for said ionic entity, characterized in that, after gelling by said ionic gelling entity, the proportion of ionic gelling entity is brought to a level which is less than 0.75 times that of the maximum proportion corresponding to said saturation.

13. The method according to claim 12, characterized in that the proportion of ionic gelling entity is brought to a level which is less than 0.5 times that of the maximum proportion corresponding to the above-mentioned saturation, and particularly preferably between 0.005 times and 0.05 times that of said maximum proportion.

14. The method according to claim 12 or 13, characterized in that the above-mentioned proportion of ionic entity in the gel formed by the gelling of the ionically gellable material is reduced by ion exchange, in particular with protons, for example by means of an aqueous solution of an acid which is brought into contact with the above-mentioned gel so that ion exchange takes place between said ionic entity and the proton.

15. The method according to claim 14, characterized in that the proportion of ionic entity is reduced with an aqueous solution of an acid whose pH is between 1 and 3.5 and preferably between 2.5 and 3.2.

16. The method according to claim 14 or 15, characterized in that an acid acceptable in foodstuffs, such as lactic acid, is used.

17. The method according to claim 14, 15 or 16, characterized in that the acid used is capable of forming a complex with the ionic gelling entity, so as to accelerate the reduction of the proportion of ionic gelling entity, for example when the ionic gelling entity is a calcium ion, the acid used is an organic diacid in which said acid group preferably occupies the 1 and 4 positions, such as tartaric acid.

18. The method according to one of claims 12 to 17, characterized in that the ionically gellable material constitutes a material compatible with a fermentation medium, in particular in the field of oenology and preferably consisting of wine, for the production of sparkling wines and especially champagne.

19. The method according to one of claims 12 to 18, characterized in that the ionically gellable material constitutes a material for the inclusion of micro-organisms, especially fermentation micro-organisms such as yeasts, or macromolecules such as enzymes, so as to obtain a gelled biocatalyst which is deficient in ionic crosslinking entity and has an affinity for ions, in particular calcium ions.

20. The method according to one of claims 12 to 19, characterized in that the gel prepared is a gel with a double layer structure comprising a core in which micro-organism cells are included, and an outer layer devoid of said cells.

21. The method according to one of claims 19 and 20, characterized in that the proportion of ionic gelling entity is lowered at a temperature of between 4°C and 10°C, preferably at about 4°C.

22. The method according to one of claims 19 to 21, characterized in that, during the operation of lowering the proportion of ionic gelling entity, nutrient substrate is introduced into the medium containing the gel or biocatalyst, in a quantity which is just sufficient to ensure the viability of the micro-organisms, such as the yeasts, included in said gel or biocatalyst.

23. The method according to claim 22, characterized in that the above-mentioned micro-organism is Saccharomyces cerevisiae and the quantity of nutrient substrate introduced into this medium is about 0.4 g of sucrose per hour for 300.10⁶ cells.

24. The method according to one of claims 19 to 23, characterized in that the pH of the above-mentioned acid solution is maintained at a value of at least 2.7 when the gel contains micro-organism cells, such as yeast cells, so as to preserve the activity of said cells.

25. The method according to one of claims 18 to 24, characterized in that the proportion of ionic gelling entity is less than or equal to about 0.30 times the maximum proportion corresponding to saturation.

26. The method according to one of claims 12 to 24 , characterized in that, after the step for depletion of the ionic gelling entity, a further ion exchange can be carried out in order to introduce a metal ion for a particular purpose, such as enzymic activation or the recognition, binding or purification of an organic material such as proteins or amino acids.

27. The method according to claim 26, characterized in that this metal ion is preferably selected from the group consisting of magnesium, manganese, zinc, potassium, iron, copper, calcium, cobalt and molybdenum.

28. Use of the gel such as defined in anyone of claims 1 to 11, or such as obtained by the method according to any one of claims 12 to 27, as a material for trapping ions, in particular cations.

29. The use of the gel, according to claim 28, in particular in the form of beads, in the food industry, especially in the fruit juice industry and in oenology, for preventing or reducing the risks of the precipitation of crystals such as potassium bitartrate and calcium tartrate.

30. The use according to claim 28 or 29, characterized in that an ionotropic gel, such as calcium alginate, which is deficient in ionic gelling entity, preferably in the form of beads, is used in a fermentation process, in particular in a bottle according to the so-called "champagne method", which consists of the secondary ferment of a wine, such as a champagne wine, after the addition of sugar to give a sparkling wine.

31. The use according to claim 30, characterized in that the gel contains yeasts such as Saccharomyces cerevisiae or Saccharomyces bayanus.

32. The use according to claim 31, characterized in that the quantity of calcium alginate gel containing yeasts, preferably in the form of beads, which is introduced into each 75 cl bottle for the fermentation process is generally about 4 ml for a concentration of about 3.10⁸ yeast cells per millilitre of gel.

33. The use according to one of claims 30 to 32, characterized in that calcium alginate gel which is not deficient in calcium ions and includes yeasts is used in conjunction with gel which does not incorporate yeast and is deficient in calcium ions, the sole purpose of the latter gel being to trap the undesirable cations such as the potassium ions and the calcium ions.

34. The use according to one of claims 29 to 33, characterized in that the proportion of ionic gelling entity is less than or equal to about 0.30 times the maximum proportion corresponding to saturation.

35. The use of the ionotropic gel according to one of claims 1 to 11, in particular in the form of beads or bound to an appropriate support such as a grid or filament, in processes for the removal of heavy metals such as lead, barium, cobalt, iron, manganese and copper.

36. The use according to claim 35, characterized in that the gel is used, for example as column packing, for the continuous treatment of water, especially the treatment of urban and industrial effluents.

37. The use of the gel such as defined in one of claims 1 to 11, or such as prepared by the method according to one of claims 12 to 27, in an enzymic process, thus making it possible to regulate the enzymic activity, in which case said gel comprises a determined proportion of enzymic activating ions selected in particular from the group consisting of magnesium, manganese, zinc, potassium, iron, copper, calcium, cobalt and molybdenum.

38. The use of the gel such as defined in one of claims 1 to 11, or such as prepared by the method according to one of claims 12 to 27, in a process for the binding or purification of an organic material, in particular proteins or amino acids, or in a process for the recognition of such proteins or amino acids.

39. The use according to claim 38, characterized in that the ionotropic gel contains a predetermined quantity of binding metal ions selected in particular from the group consisting of magnesium, manganese, zinc, potassium, iron, copper, calcium, cobalt and molybdenum, which make it possible to bind the organic material, in particular proteins or amino acids.

## Patentansprüche

1. Festes ionotropes Gel, insbesondere in Form von Kügelchen oder fixiert auf einem geeigneten Träger, wie einem Netz oder Filament, das aus einem mit einem ionischen Geliergebilde gelierbaren Material gebildet ist, dadurch gekennzeichnet, daß das Gel an ionischem Geliergebilde defizient ist und ionische Fixierungsstellen aufweist, die aus der Abwesenheit des ionischen Gebildes resultieren, und die ihm auf diese Weise eine Affinität für Ionen verleihen, welche sich am Gel an den Fixierungsstellen fixieren können, die nicht vom ionischen Geliergebilde besetzt sind, wobei der Gehalt an ionischem Geliergebilde weniger als das 0,75-fache des der Sättigung im Gel entsprechenden maximalen Gehalts beträgt.

2. Ionotropes Gel nach Anspruch 1, dadurch gekennzeichnet, daß der Gehalt an ionischem Geliergebilde weniger als das 0,5-fache und vorzugsweise zwischen dem 0,005-fachen und 0,05-fachen des der Sättigung im Gel entsprechenden maximalen Gehalts der Fixierungsstellen des ionischen Geliergebildes beträgt.

3. Ionotropes Gel nach Anspruch 1 oder 2, dadurch gekennzeichnet, daß das gelierbare Material fließfähig sein kann, und vorteilhaft in Form von Tröpfchen verwendet werden kann, die geliert werden, indem sie mit einer wässerigen Lösung, die das ionische Geliergebilde enthält, in Berührung gebracht wird.

4. Ionotropes Gel nach einem der Ansprüche 1 bis 3, dadurch gekennzeichnet, daß das gelierbare Material ausgewählt ist aus der Gruppe bestehend aus wasserlöslichen Salzen von Alginsäure und Pektinsäure, insbesondere Salzen von Alkalimetallen, wie Natrium oder Kalium, oder Ammoniumsalz, Carrageenan, insbesondere in Form von Iota und Kappa, Chitosan und Carboxymethylcellulose.

5. Ionotropes Gel nach einem der Ansprüche 1 bis 4, dadurch gekennzeichnet, daß das gelierbare ionische Material ein durch Calciumionen gelierbares Material ist, wobei das ionische Geliergebilde auf diese Weise aus Calciumionen besteht, um so ein an Calciumionen verarmtes, kationenanziehendes ionotropes Gel zu erhalten.

6. Ionotropes Gel nach Anspruch 5, dadurch gekennzeichnet, daß es aus Calciumalginat besteht, dessen Calciumionen-Gehalt weniger als 1,5 mg/g feuchtes Gel, vorzugsweise weniger als 1 mg/g und bevorzugt zwischen 0,01 mg/g und 0,1 mg/g feuchtes Gel beträgt.

7. Ionotropes Gel nach einem der Ansprüche 1 bis 6, dadurch gekennzeichnet, daß der an ionischem Geliergebilde defiziente Gehalt durch Ionenaustausch mit Protonen erhalten wird, beispielsweise durch eine wässerige Lösung einer Säure, die mit dem Gel in Berührung gebracht wird, um einen Ionenaustausch zwischen dem ionischen Gebilde und den Protonen zu bewirken.

8. Ionotropes Gel nach einem der Ansprüche 1 bis 7, dadurch gekennzeichnet, daß das gelierbare ionische Material ein Material für den Einschluß von Mikroorganismen ist, insbesondere Fermentationsmikroorganismen, wie Hefen, oder Makromolekülen, wie Enzymen, um einen gelierten Biokatalysator zu erhalten, der an ionischem Vernetzungsgebilde defizient ist und Ionen, insbesondere Calciumionen, anzieht.

9. Ionotropes Gel nach Anspruch 8, dadurch gekennzeichnet, daß das ionisch gelierbare Einschlußmaterial ein geeignetes Material ist, das mit einem Fermentationsmedium, insbesondere auf dem Gebiet der Önologie, kompatibel ist, und vorzugsweise aus Wein besteht, um Schaumwein und insbesondere Champagner herzustellen.

10. Ionotropes Gel nach Anspruch 9, dadurch gekennzeichnet, daß das Einschlußmaterial ausgewählt ist aus der Gruppe bestehend aus Alkali- oder Ammoniumsalzen von Algin- oder Pektinsäure, vorzugsweise Natrium- oder Kaliumalginat.

11. Ionotropes Gel nach einem der Ansprüche 9 oder 10, dadurch gekennzeichnet, daß der Gehalt an ionischem Geliergebilde kleiner oder gleich ist dem etwa 0,30-fachen des der Sättigung entsprechenden maximalen Gehalts.

12. Verfahren zur Herstellung eines ionotropen festen Gels, das defizient ist an ionischem Geliergebilde, welches Verfahren die Gelierung eines ionisch gelierbaren Materials umfaßt, das Fixierungsstellen oder Vernetzungsstellen aufweist, an denen sich das ionische Gebilde fixiert, wobei auf diese Weise im so gebildeten Gel die Sättigung der Fixierungsstellen des ionischen Gebildes bewirkt wird, dadurch gekennzeichnet, daß nach der Gelierung durch das ionische Geliergebilde der Gehalt an ionischem Geliergebilde auf einen Wert von weniger als dem 0,75-fachen des der Sättigung entsprechenden maximalen Gehalts geführt wird.

13. Verfahren nach Anspruch 12, dadurch gekennzeichnet, daß der Gehalt an ionischem Geliergebilde auf einen Wert von weniger als dem 0,5-fachen des der Sättigung entsprechenden maximalen Gehalts und bevorzugt auf einen Wert zwischen dem 0,005-fachen und 0,05-fachen des maximalen Gehalts geführt wird.

14. Verfahren nach Anspruch 12 oder 13, dadurch gekennzeichnet, daß die Verringerung des Gehalts an ionischem Gebilde des durch die Gelierung eines ionisch gelierbaren Materials gebildeten Gels durch Ionenaustausch durchgeführt wird, insbesondere mit Protonen, beispielsweise mit einer wässerigen Lösung einer Säure, die mit dem Gel in Berührung gebracht wird, um einen Ionenaustausch zwischen dem ionischen Gebilde und den Protonen zu bewirken.

15. Verfahren nach Anspruch 14, dadurch gekennzeichnet, daß die Verringerung des Gehalts an ionischem Gebilde mit einer wässerigen Lösung einer Säure durchgeführt wird, deren pH zwischen 1 und 3,5 liegt, und vorzugsweise zwischen 2,5 und 3,2 liegt.

16. Verfahren nach Anspruch 14 oder 15, dadurch gekennzeichnet, daß eine für Nahrungsmittel annehmbare Säure, wie Milchsäure, verwendet wird.

17. Verfahren nach Anspruch 14, 15 oder 16, dadurch gekennzeichnet, daß eine Säure verwendet wird, die einen Komplex mit dem ionischen Geliergebilde bilden kann, um die Verringerung des Gehalts an ionischem Geliergebilde zu beschleunigen, wobei, wenn das ionische Geliergebilde beispielsweise Calciumionen sind, eine organische Disäure verwendet wird, deren Säurefunktion vorzugsweise Stellung 1 und 4 besetzt, wie Weinsäure.

18. Verfahren nach einem der Ansprüche 12 bis 17, dadurch gekennzeichnet, daß das ionisch gelierbare Material ein Material ist, das mit einem Fermentationsmedium, insbesondere auf dem Gebiet der Önologie, kompatibel ist, und vorzugsweise aus Wein besteht, um Schaumweine und insbesondere Champagner herzustellen.

19. Verfahren nach einem der Ansprüche 12 bis 18, dadurch gekennzeichnet, daß das ionisch gelierbare Material ein Material für den Einschluß von Mikroorganismen ist, insbesondere Fermentationsmikroorganismen, wie Hefen, oder Makromolekülen, wie Enzymen, um einen gelierten Biokatalysator zu erhalten, der an ionischem Vernetzungsgebilde defizient ist und Ionen, insbesondere Calciumionen, anzieht.

20. verfahren nach einem der Ansprüche 12 bis 19, dadurch gekennzeichnet, daß ein Gel mit einer Doppelschichtstruktur hergestellt wird, die einen Kern, in dem Zellen von Mikroorganismen eingeschlossen sind, und eine äußere Schicht ohne diese Zellen umfaßt.

21. Verfahren nach einem der Ansprüche 19 und 20, dadurch gekennzeichnet, daß die Senkung des Gehalts an ionischem Geliergebilde bei einer Temperatur zwischen 4°C und 10°C, vorzugsweise bei etwa 4°C, durchgeführt wird.

22. Verfahren nach einem der Ansprüche 19 bis 21, dadurch gekennzeichnet, daß beim Vorgang der Senkung des Gehalts an ionischem Geliergebilde ein Nährstoffsubstrat dem das Gel enthaltenden Medium oder dem Biokatalysator in einer Menge zugeführt wird, die gerade notwendig ist, um die Lebensfähigkeit von im Gel oder Biokatalysator eingeschlossenen Mikroorganismen, wie Hefen, sicherzustellen.

23. Verfahren nach Anspruch 22, dadurch gekennzeichnet, daß der Mikroorganismus Saccharomyces cerevisiae ist, wobei die Menge an dem Medium zugeführtem Nährstoffsubstrat etwa 0,4 g Saccharose pro Stunde für 300 x 10⁶ Zellen beträgt.

24. Verfahren nach einem der Ansprüche 19 bis 23, dadurch gekennzeichnet, daß der pH der sauren Lösung auf einem Wert von zumindest 2,7 gehalten wird, wenn das Gel Zellen von Mikroorganismen, wie Hefen, enthält, um die Aktivität dieser Zellen aufrechtzuerhalten.

25. Verfahren nach einem der Ansprüche 18 bis 24, dadurch gekennzeichnet, daß der Gehalt an ionischem Geliergebilde kleiner oder gleich ist dem etwa 0,30-fachen des der Sättigung entsprechenden maximalen Gehalts.

26. Verfahren nach einem der Ansprüche 12 bis 24, dadurch gekennzeichnet, daß nach dem Schritt der Verarmung an ionischem Geliergebilde ein neuer Ionenaustausch, um Metallionen einzuführen, für eine bestimmte Verwendung durchgeführt werden kann, wie die Enzymaktivierung oder Erkennung, die Fixierung oder Reinigung von einem organischen Material, wie Proteinen oder Aminosäuren.

27. Verfahren nach Anspruch 26, dadurch gekennzeichnet, daß diese Metallionen vorzugsweise ausgewählt werden aus der Gruppe bestehend aus Magnesium, Mangan, Zink, Kalium, Eisen, Kupfer, Calcium, Kobalt und Molybdän.

28. Verwendung eines Gels, wie oben in einem der Ansprüche 1 bis 11 definiert, oder wie durch das Verfahren nach einem der Ansprüche 12 bis 27 erhalten, als Material zum Abfangen von Ionen, insbesondere Kationen.

29. Verwendung eines Gels nach Anspruch 28, insbesondere in Form von Kügelchen, in der Nahrungsmittelindustrie, insbesondere in der Fruchtsaftindustrie und Önologie, zur Verhinderung oder Verringerung von Risken einer Ausfällung von Kristallen, wie Kaliumbitartrat und Calciumtartrat.

30. Verwendung nach Anspruch 28 oder 29, dadurch gekennzeichnet, daß ein ionotropes Gel, wie Calciumalginat, das an ionischem Geliergebilde defizient ist, vorzugsweise in Form von Kügelchen in einem Moussierverfahren, insbesondere in einer Flasche gemäß der als "Champagner-Methode" bekannten Methode, verwendet wird, die aus der zweiten Fermentation von Wein, wie Champagnerwein, nach dem Zusatz von Zucker besteht, um Schaumwein zu erhalten.

31. Verwendung nach Anspruch 30, dadurch gekennzeichnet, daß das Gel Hefen, wie Saccharomyces cerevisiae oder Saccharomyces bayanus, enthält.

32. Verwendung nach Anspruch 31, dadurch gekennzeichnet, daß die Menge an Hefen enthaltendem Calciumalginat-Gel, die vorzugsweise in Form von Kügelchen pro 75 cl Flasche eingeführt wird, im Moussierverfahren allgemein etwa 4 ml für eine Konzentration von etwa 3 x 10⁸ Hefezellen pro ml Gel beträgt.

33. Verwendung nach einem der Ansprüche 30 bis 32, dadurch gekennzeichnet, daß das Calciumalginat-Gel, das nicht an Calciumionen defizient ist, und Hefen enthält, zusammen mit einem Gel verwendet wird, das keine Hefe enthält, und an Calciumionen defizient ist, wobei letzteres die einzige Funktion hat, die unerwünschten Kationen, wie Kaliumionen und Calciumionen, abzufangen.

34. Verwendung nach einem der Ansprüche 29 bis 33, dadurch gekennzeichnet, daß der Gehalt an ionischem Geliergebilde kleiner oder gleich ist dem etwa 0,30-fachen des der Sättigung entsprechenden maximalen Gehalts.

35. Verwendung eines ionotropen Gels nach einem der Ansprüche 1 bis 11, insbesondere in Form von Kügelchen oder fixiert auf einem geeigneten Träger, wie einem Netz oder Faden, in Verfahren zur Eliminierung von Schwermetallen, wie Blei, Barium, Kobalt, Eisen, Mangan und Kupfer.

36. Verwendung nach Anspruch 35, dadurch gekennzeichnet, daß das Gel beispielsweise zum Füllen von Säulen zur kontinuierlichen Behandlung von Abwässern, insbesondere jener von städtischen und Industrieabwässern, verwendet wird.

37. Verwendung eines Gels, wie in einem der Ansprüche 1 bis 11 definiert oder wie durch das Verfahren nach einem der Ansprüche 12 bis 27 hergestellt, in einem enzymatischen Verfahren, wobei auf diese Weise die Steuerung der Enzymaktivität ermöglicht wird, welches Gel so einen bestimmten Gehalt an Enzymaktivatorionen aufweist, insbesondere ausgewählt aus der Gruppe bestehend aus Magnesium, Mangan, Zink, Kalium, Eisen, Kupfer, Calcium, Kobalt, Molybdän.

38. Verwendung eines Gels, wie in einem der Ansprüche 1 bis 11 definiert oder wie durch das Verfahren nach einem der Ansprüche 12 bis 27 hergestellt, in einem Verfahren zur Fixierung oder Reinigung von einem organischen Material, insbesondere Proteinen oder Aminosäuren, oder in einem Verfahren zur Erkennung derartiger Proteine oder Aminosäuren.

39. Verwendung nach Anspruch 38, dadurch gekennzeichnet, daß das ionotrope Gel eine vorherbestimmte Menge an Metallionen zur Fixierung enthält, welche ausgewählt sind aus der Gruppen bestehend aus Magnesium, Mangan, Zink, Kalium, Eisen, Kupfer, Calcium, Kobalt, Molybdän, und welche die Fixierung des organischen Materials, insbesondere der Proteine oder Aminosäuren, ermöglichen.
